# DEMANDE DE BREVET EUROPEEN

(11) **EP 1 234 570 A1**
(43) Date de publication de la demande: **28.08.2002**
(21) Numéro de dépôt: 02290412.2
(22) Date de dépôt: 20.02.2002
(51) Int. Cl.: A61K 7/48

(54) **Utilisation cosmétique pour le traitement des peaux grasses d'une composition matifiante**

(30) Priorité: 22.02.2001 FR 0102417
(71) Demandeur: L'OREAL, 75008 Paris (FR)
(72) Inventeur: Chevalier, Véronique, 94440 Villecresnes (FR); Quest, Mélanie, 75005 Paris (FR)
(74) Mandataire: Catherine, Alain

(57) **Abrégé**

L'invention concerne l'utilisation cosmétique pour le traitement des peaux d'une composition cosmétique matifiante contenant, dans un milieu physiologiquement acceptable, une quantité effective de polytétrafluoroéthylène (PTFE). Selon l'invention, le polytétrafluoroéthylène (PTFE) est sous forme de particules en dispersion dans un milieu aqueux, les particules ayant une dimension moyenne en nombre allant d'environ 0,1 à 30µm, et de préférence de 0,5 à 15µm.

## Description

La présente invention concerne l'utilisation cosmétique pour le traitement des peaux grasses d'une composition cosmétique matifiante contenant, dans un milieu physiologiquement acceptable, du polytétrafluoroéthylène (PTFE) sous forme de particules en dispersion dans un milieu aqueux.

Par définition, un produit matifiant est un produit qui empêche la peau de briller et qui unifie le teint. Les compositions de soin de la peau ou de maquillage ayant des propriétés matifiantes sont généralement utilisées pour résoudre les problèmes de brillance occasionnés par un excès de sébum, et pour améliorer la tenue du maquillage à long terme, le maquillage ayant tendance à se dégrader visuellement au cours de la journée. Ces compositions donnent un aspect mat à la peau, résultant d'un pouvoir diffusant de la lumière à la surface de la peau. Elles peuvent aussi être utilisées pour estomper les défauts de la peau, tels que les microreliefs, les rides, les ridules, les pores ou les variations de couleur.

De façon générale, les compositions cosmétiques matifiantes sont formulées à l'aide de charges minérales (ZnO, SiO₂, ...) enrobées ou non, et d'amidon. Ces particules sont très connues et très employées mais présentent l'inconvénient de sédimenter. Il subsiste donc le besoin d'une composition cosmétique matifiante fluide ayant un seuil de viscosité élevé.

La demanderesse a maintenant trouvé une composition surmontant les inconvénients de l'art antérieur.

La demanderesse a découvert de manière surprenante que l'utilisation de polytétrafluoroéthylène (PTFE) sous forme de particules submicroniques en dispersion dans un milieu aqueux, conférait à cette composition, après application sur la peau, un aspect mat de façon prolongée dans le temps, tout en étant confortable et non desséchante.

L'homme de l'art connaît l'utilisation de PTFE dans des compositions cosmétiques.

En effet, la demande de brevet internationale WO/9103228 décrit un produit cosmétique comprenant un polymère fibrillaire ajouté dans une poudre compactée, le polymère fibrillaire pouvant être du polytétrafluoroéthylène (PTFE) ou du polypropylène.

De même, la demande de brevet européen EP-A-0997134 décrit l'utilisation d'une phase particulaire dans une composition cosmétique pour masquer les imperfections de la peau. Cette phase particulaire comprend des particules de nature polymérique, de dimensions moyennes allant de 5 à 100 µm. Les particules peuvent être notamment constituées de polytétrafluoroéthylène (PTFE).

Enfin, le document EP-A-0502769 décrit des compositions matifiantes apportant une couche translucide et un aspect naturel à la peau maquillée. Il s'agit de dispersions de particules solides, sphériques ou sphéroïdales dans un liant gras, ayant des dimensions allant de 0,5 à 25 µm et constituées d'un matériau compatible avec une application topique de la composition, comme par exemple le PTFE.

Cependant, rien n'est dit dans ces documents concernant l'effet matifiant apporté par des dispersions aqueuses de polytétrafluoroéthylène (PTFE).

La présente invention a donc pour objet l'utilisation cosmétique pour le traitement des peaux grasses d'une composition cosmétique matifiante, contenant dans un milieu physiologiquement acceptable, une quantité efficace de polytétrafluoroéthylène (PTFE), le polytétrafluoroéthylène étant sous forme de particules en dispersion dans un milieu aqueux et ayant une dimension moyenne en nombre allant d'environ 0,1 à 30 µm, et de préférence de 0,5 à 15 µm.

Par "composition matifiante", on entend au sens de la demande une composition permettant de donner un aspect mat à la peau.

On entend par "aspect mat" de la peau, un aspect non brillant, avec un teint unifié.

On entend par "milieu physiologiquement acceptable" dans la composition de l'invention, un milieu non toxique et susceptible d'être appliqué sur la peau (y compris l'intérieur des paupières) ou les lèvres d'êtres humains.

Par milieu aqueux, on entend, au sens de la demande, aussi bien un milieu contenant seulement de l'eau qu'un milieu comprenant de l'eau et un solvant hydrosoluble tel qu'un alcool inférieur comportant 1 à 6 atomes de carbone ou un glycol.

Par quantité efficace, on entend une quantité suffisante pour atteindre le but recherché, c'est-à-dire l'effet matifiant. Cette quantité dépend de la dispersion de PTFE utilisée et des autres composés de la composition.

Le polytétrafluoroéthylène (PTFE) utilisé dans la composition de l'invention n'est pas un polymère filmogène, ce qui signifie que le PTFE ne forme pas un film après évaporation de l'eau de la dispersion, mais que les particules de copolymère contenant de l'air restent à la surface du support (en particulier de la peau) et diffusent la lumière dans toutes les directions.

Comme dispersions de polytétrafluoroéthylène (PTFE), utilisables dans la composition selon l'invention, on peut citer par exemple celles commercialisées sous les dénominations :
- X-6687® (poudre de polytétrafluoroéthylène en dispersion à 50% - taille 0,7 µm), par la société SHAMROCK,
- Hydropure 9174C® (poudre de polytétrafluoroéthylène en dispersion aqueuse - taille 3 à 4 µm) par la société SHAMROCK, et
- Fluotron 200® (poudre de polytétrafluoroéthylène en dispersion aqueuse à 40% - taille 12 µm) par la société CARROLL SCIENTIFIC.

Le PTFE est généralement présent dans la composition en une quantité de matière active allant de 0,1 à 30% en poids, de préférence de 0,15 à 15% en poids et mieux de 0,5 à 5% en poids par rapport au poids total de la composition.

La composition peut se présenter sous forme d'une composition aqueuse ou sous forme d'une émulsion huile-dans-eau (H/E) ou eau-dans-huile (E/H) ou d'une émulsion multiple. Selon un mode préféré de l'invention, elle se présente sous forme d'une émulsion notamment huile-dans-eau (H/E) ou eau-dans-huile (E/H) ou multiple (E/H/E ou H/E/H).

On entend ici par "émulsion" aussi bien des dispersions sans émulsionnants que des dispersions comportant des émulsionnants ou encore des dispersions stabilisées par des particules solides ou par des sphérules lipidiques de type ionique ou non ionique.

Pour une utilisation pour les peaux grasses, on préfèrera avoir une émulsion huile-dans-eau (H/E), dont la phase aqueuse externe apporte un effet de fraîcheur.

Quand la composition est une émulsion, elle comprend une phase huileuse.

La nature de la phase huileuse de la composition de l'invention n'est pas critique et peut être constituée de tous les corps gras et notamment les huiles, classiquement utilisés dans le domaine cosmétique

La phase huileuse comprend au moins une huile.

Parmi les huiles utilisables dans la composition de l'invention, on peut notamment citer par exemple les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkylbenzoates), les huiles de silicones volatiles ou non volatiles telles que les polydiméthylsiloxanes (PDMS) et les cyclodiméthylsiloxanes ou cyclométhicones, notamment le cyclohexadiméthylsiloxane et le cyclopentadiméthylsiloxane, et les huiles fluorées ou fluorosiliconées, et leurs mélanges.

Les autres corps gras susceptibles d'être présents dans la phase huileuse peuvent être par exemple les acides gras, les alcools gras tels que l'alcool stéarylique, l'alcool cétylique et l'alcool cétéarylique, les cires, et leurs mélanges.

Dans les compositions de l'invention sous forme d'émulsion, la phase aqueuse de la composition peut être présente à raison de 1 à 80% en poids, et de préférence à raison de 30 à 70% en poids par rapport au poids total de la composition, et la phase huileuse peut être présente à raison de 5 à 70% en poids, et de préférence à raison de 10 à 50% en poids par rapport au poids total de la composition.

Les émulsions peuvent contenir au moins un émulsionnant choisi parmi les émulsionnants amphotères, anioniques, cationiques ou non ioniques, utilisés seuls ou en mélange.

Les émulsionnants sont choisis de manière appropriée suivant l'émulsion à obtenir : émulsions eau-dans-l'huile (E/H) ou huile-dans-eau (H/E).

Pour les émulsions huile-dans-eau (H/E), on peut citer par exemple les émulsionnants suivants:
- comme émulsionnants amphotères, les N-acyl-aminoacides tels que les N-alkylaminoacétates et le cocoamphodiacétate disodique et les oxydes d'amines tels que l'oxyde de stéaramine;
- comme émulsionnants anioniques, les acylglutamates tels que le "disodium hydrogenated tallow glutamate" (Amisoft HS-21® commercialisé par la société AJINOMOTO) ; les acides carboxyliques et leurs sels tels que le stéarate de sodium ; les esters phosphoriques et leurs sels tels que le "DEA oleth-10 phosphate" ; les sulfosuccinates tels que le "Disodium PEG-5 citrate lauryl sulfosuccinate" et le "Disodium ricinoleamido MEA sulfosuccinate" ;
- comme émulsionnants cationiques, les sels d'alkylimidazolidinium tels que l'éthosulfate d'isostéaryléthylimidonium ; les sels d'ammonium tels que le chlorure de N,N,N-triméthyl-1-docosanaminium (Behentrimonium chloride) ;
- comme émulsionnants non ioniques, les esters et éthers d'oses tels que le stéarate de sucrose, le cocoate de sucrose, et le mélange de stéarate de sorbitan et de cocoate de sucrose commercialisé par la société ICI sous la dénomination Arlatone 2121® ; les esters de polyol, notamment de glycérol ou de sorbitol, tels que le stéarate de glycéryle, le stéarate de polyglycéryl-2, le stéarate de sorbitan ; les éthers de glycérol ; les éthers oxyéthylénés et/ou oxypropylénés, tels que l'éther oxyéthyléné, oxypropyléné de l'alcool laurique à 25 groupes oxyéthylénés et 25 groupes oxypropylénés (nom CTFA "PPG-25 laureth-25") et l'éther oxyéthyléné du mélange d'alcools gras en C₁₂-C₁₅ comportant 7 groupes oxyéthylénés (nom CTFA " C₁₂-C₁₅ Pareth-7") ; les polymères d'éthylène glycol, tels que le PEG-100.

On peut utiliser un ou plusieurs de ces émulsionnants.

Pour les émulsions eau-dans-huile (E/H), on peut citer comme exemple d'émulsionnants, les esters gras de polyol, notamment de glycérol ou de sorbitol, et notamment les esters isostéariques, oléiques et ricinoléiques de polyol, tels que le mélange de petrolatum, d'oléate de polyglycéryl-3, d'isostéarate de glycéryle, l'huile de ricin hydrogénée et d'ozokérite, vendu sous la dénomination Protegin W® par la société GOLDSCHMIDT, l'isostéarate de sorbitan, le diisostéarate de polyglycéryle, le sesqui-isostéarate de polyglycéryle-2 ; les esters et éthers d'oses tels que le "Methyl glucose dioléate" ; les esters gras tels que le lanolate de magnésium ; les diméthicone copolyols et alkylsdiméthicone copolyols tels que le Laurylmethicone copolyol vendu sous la dénomination Dow Corning 5200 Formulation Aid® par la société DOW CORNING et le Cetyl dimethicone copolyol vendu sous la dénomination Abil EM 90® par la société GOLDSCHMIDT; et leurs mélanges.

Les émulsionnants peuvent être introduits tels quels ou sous forme de mélange avec d'autres émulsionnants et/ou avec d'autres composés tels que des alcools gras ou des huiles.

La composition de l'invention peut contenir en plus des adjuvants classiques tels que des colorants hydrosolubles ou liposolubles, des pigments, des parfums, des conservateurs, des filtres solaires chimiques ou physiques, des séquestrants (EDTA), des actifs liposolubles ou hydrosolubles, des hydratants tels que les polyols et notamment la glycérine, des ajusteurs de pH (acides ou base), et des charges.

Ces adjuvants peuvent être présents dans des quantités allant de préférence de 0,01 à 20% en poids par rapport au poids total de la composition.

Ces adjuvants ainsi que leurs concentrations doivent être tels qu'ils ne modifient pas la propriété recherchée pour la composition.

Comme filtres solaires chimiques utilisables dans la composition de l'invention, la composition de l'invention peut comprendre tous les filtres UVA et UVB utilisables dans le domaine cosmétique.

Comme filtres UVB, on peut citer par exemple:
1. les dérivés de l'acide salicylique, en particulier le salicylate d'homomenthyle et le salicylate d'octyle ;
2. les dérivés de l'acide cinnamique, en particulier le p-méthoxycinnamate de 2-éthylhexyle, commercialisé par la société Givaudan sous la dénomination Parsol MCX® ;
3. les dérivés de β,β'-diphénylacrylate liquides, en particulier l'α-cyano-α,β'diphénylacrylate de 2-éthylhexyle ou octocrylène, commercialisé par la société BASF sous la dénomination UVINUL N539® ;
4. les dérivés de l'acide p-aminobenzoïque ;
5. le 4-méthyl benzylidène camphre commercialisé par la société Merck sous la dénomination EUSOLEX 6300® ;
6. l'acide 2-phénylbenzimidazole 5-sulfonique commercialisé sous la dénomination EUSOLEX 232® par la société Merck ;
7. les dérivés de 1,3,5-triazine, en particulier:
   - la 2,4,6-tris[p-(2'-éthylhexyl-1'-oxycarbonyl)anilino]-1,3,5-triazine commercialisé par la société BASF sous la dénomination UVINUL T150®, et
   - le dioctyl butamido triazone commercialisé par la société Sigma 3V sous la dénomination UVASORB HEB® ;
8. les mélanges de ces filtres.

Comme filtres UVA, on peut citer par exemple:
1. les dérivés de dibenzoylméthane, en particulier le 4-(ter.-butyl) 4'-méthoxy dibenzoylméthane commercialisé par la société Givaudan sous la dénomination PARSOL 1789® ;
2. l'acide benzène 1,4[di(3-méthylidènecampho-10-sulfonique)] éventuellement sous forme partiellement ou totalement neutralisée, commercialisé sous la dénomination MEXORYL SX® par la société Chimex.
3. les dérivés de benzophénone, par exemple :
   - la 2,4-dihydroxybenzophénone (benzophénone-1),
   - la 2,2',4,4'-tétra-hydroxybenzophénone (benzophénone-2),
   - la 2-hydroxy-4-méthoxy-benzophénone (benzophénone-3), commercialisé sous la dénomination UVINUL M40® par la société BASF,
   - l'acide 2-hydroxy-4-méthoxy-benzophénone-5-sulfonique (benzo-phénone-4) ainsi que sa forme sulfonate (benzophénone-5), commercialisé par la société BASF sous la dénomination UVINUL MS40®,
   - la 2,2'-dihydroxy-4,4'-diméthoxy-benzophénone (benzophénone-6),
   - la 5-chloro-2-hydroxybenzophénone (benzophénone-7),
   - la 2,2'-dihydroxy-4-méthoxy-benzophénone (benzophénone-8) ;
   - le sel disodique du diacide 2,2'-dihydroxy-4,4'-diméthoxybenzophénone-5,5'-disulfonique (benzophénone-9),
   - la 2-hydroxy-4-méthoxy-4'-méthyl-benzophénone (benzophénone-10),
   - la benzophénone-11 ;
   - la 2-hydroxy-4-(octyloxy)benzophénone (benzophénone-12);
4. les dérivés silane ou les polyorganosiloxanes à groupement benzophénone ;
5. les anthranilates, en particulier l'anthranilate de menthyle commercialisé par la société Haarman & Reiner sous la dénomination NEO HELIOPAN MA® ;
6. les composés comportant par molécule au moins deux groupes benzoazolyle ou au moins un groupe benzodiazolyle, en particulier l'acide 1,4-bis-benzimidazolyl-phenylène-3,3',5,5'-tétrasulfonique ainsi que ses sels commercialisés par la société Haarman & Reimer ;
7. les dérivés siliciés de benzimidazolyl-benzazoles N-substitués ou de benzofuranyl-benzazoles, et en particulier :
   - le 2-[1-[3-[1,3,3,3-tétraméthyl-1-[(triméthylsilyl)oxy]disiloxanyl] propyl]-1H-benzimidazol-2-yl]-benzoxazole,
   - le 2-[1-[3-[1,3,3,3-tétraméthyl-1-[(triméthylsilyl)oxy]disiloxanyl] propyl]-1H-benzimidazol-2-yl]-benzothiazole,
   - le 2-[1-(3-trimethylsilanyl-propyl)-1H-benzimidazol-2-yl]-benzoxazole,
   - le 6-méthoxy-1,1'-bis-(3-triméthylsilanyl-propyl)-1H,1'H-[2,2']bi benzimidazolyl-benzoxazole,
   - le 2-[1-(3-triméthylsilanyl-propyl)-1H-benzimidazol-2-yl]benzothiazole,
   qui sont décrits dans la demande de brevet EP-A-1.028.120 ;
8. les dérivés de triazine, et en particulier la 2,4-bis {[4-(2-éthyl- hexyloxy)-2-hydroxy]-phényl}-6-(4-méthoxyphényl)-1,3,5-triazine commercialisé par la société Ciba Geigy sous la dénomination TINOSORB S®, et le 2,2'-méthylènebis-[6-(2Hbenzotriazol-2-yl)4-(1,1,3,3-tétraméthylbutyl)phénol] commercialisé par la société Ciba Geigy sous la dénomination TINOSORB M®; et
9. leurs mélanges.

On peut aussi utiliser un mélange de plusieurs de ces filtres et un mélange de filtres UVB et de filtres UVA et aussi des mélanges avec des filtres physiques.

Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm. On utilise de préférence dans la composition de l'invention des nano-pigments.

Les filtres peuvent être présents en une quantité allant de 0,01 à 20% en poids, et de préférence de 0,1 à 10% en poids par rapport au poids total de la composition.

Comme actifs, on peut citer notamment les actifs utiles pour traiter les peaux grasses, tels que les sels de zinc et en particulier le gluconate de zinc, les antibactériens comme les vitamines B3 (niacinamide) et B5 (panthénol), l'oxyde de zinc et ses dérivés, l'acide salicylique, le triclosan, le lipacide C8G® commercialisé par la société SEPPIC ou capryloylglycine, l'extrait de clou de girofle, l'octopirox, l'hexamidine, l'acide azélaïque, les actifs anti-acné et leurs mélanges.

Comme charges pouvant être utilisées dans la composition selon l'invention, on peut citer notamment la poudre de silice, le talc, les particules de polyamides et notamment celles vendues sous la dénomination ORGASOL® par la société ATOCHEM ; les poudres de polyéthylène, les microsphères à base de copolymères acryliques, telles que celles en copolymère diméthacrylate d'éthylène glycol/méthacrylate de lauryle vendues sous la dénomination POLYTRAP® par la société DOW CORNING ; les poudres expansées, telles que les microsphères creuses, et notamment, les microsphères commercialisées sous la dénomination EXPANCEL® par la société KERMANORD PLAST ou les microsphères commercialisées sous la dénomination MICROPEARL F 80 ED® par la société MATSUMOTO ; les poudres de matériaux organiques naturels, tels que les amidons de maïs, de blé ou de riz, réticulés ou non, telles que les poudres d'amidon réticulé par l'anhydride octénylglosuccinique, commercialisées sous la dénomination DRY-FLO® par la société NATIONAL STARCH ; les microbilles de résine de silicone, telles que celles commercialisées sous la dénomination TOSPEARL® par la société TOSCHIBA SILICONE ; et leurs mélanges.

Ces charges peuvent être présentes dans des quantités allant de 0 à 40% en poids, et de préférence de 0,1 à 30% en poids, et mieux de 0,5 à 10% en poids par rapport au poids total de la composition.

Par ailleurs, selon la fluidité de la composition que l'on souhaite obtenir, on peut y ajouter un ou plusieurs hydrophiles ou lipophiles, choisis par exemple parmi les argiles, les gommes polysaccharides et leurs dérivés (gomme de xanthane, carboxyméthylcellulose, hydroxypropylguar), les polymères carboxyvinyliques ou carbomers, les polyacrylamides tels que celui commercialisé sous la dénomination SEPIGEL 305® par la société SEPPIC, les polymères d'acide acrylamidométhylpropanesulfonique au moins partiellement réticulés, tels que le produit commercialisé sous la dénomination HOSTACERIN AMPS® (nom CTFA: Ammonium polyacryldiméthyltauramide) par la société HOECHST, et leurs mélanges.

Ces gélifiants sont généralement utilisés à des concentrations allant de 0,05 à 10%, de préférence 0,1 à 5% et mieux de 0,1 à 3% du poids total de la composition.

La composition selon l'invention est destiné à une application topique et trouve notamment une application dans un grand nombre de traitements cosmétiques et/ou dermatologiques de la peau et notamment en vue de lui apporter un aspect mat.

Aussi, un autre objet de l'invention consiste en un procédé de traitement cosmétique des peaux grasses comprenant sur la peau l'application d'une composition cosmétique telle que définie précédemment.

Les exemples qui suivent servent à illustrer sans toutefois présenter un caractère limitatif. Les quantités y sont données en % en poids, sauf mention contraire.

### EXEMPLE 1

### Crème matifiante: émulsion H/E

### Phase huileuse:

- Cyclohexadiméthylsiloxane 10%
- Alcool stéarylique 1%
- Stéarate de glycéryle
   (et) PEG-100 stéarate 2%

### Phase aqueuse:

- Gomme de xanthane 0,2%
- Ammonium polyacryldiméthyltauramide commercialisé par la société HOECHST sous la dénomination HOSTACERIN AMPS® 0,4%
- Hydroxyde de sodium 0,01%
- Glycérine 5%
- Aluminium starch octénylsuccinate commercialisé par la société NATIONAL STARCH sous la dénomination DRY-FLO® 3%
- PTFE en dispersion aqueuse à 40% (taille : 12 µm)
   commercialisé par la société CARROLL SCIENTIFIC sous la dénomination FLUOTRON 200® 3,75%
- Conservateurs 0,70%
- Eau qsp 100%

### Mode opératoire:

L'émulsion est préparée en ajoutant, sous agitation, la phase huileuse chauffée à 65°C à la phase aqueuse chaude.

On obtient une composition apte à matifier la peau.

### EXEMPLE 2

### Crème matifiante : émulsion H/E

### Phase huileuse

- Cyclohexadiméthylsiloxane 10%
- Alcool stéarylique 1%
- Stéarate de glycéryle
   (et) PEG-100 stéarate 2%

### Phase aqueuse:

- Gomme de xanthane 0,2%
- Ammonium polyacryldiméthyltauramide commercialisé par la société HOECHST sous la dénomination HOSTACERIN AMPS® 0,4%
- Hydroxyde de sodium 0,01%
- Glycérine 5%
- Aluminium starch octénylsuccinate commercialisé par la société NATIONAL STARCH sous la dénomination DRY-FLO® 3%
- PTFE en dispersion aqueuse à 60% (taille: 10 µm)
   commercialisé par la société SHAMROCK sous la dénomination HYDROPURE 9174C® 2,5%
- conversateurs 0,7 %
- eau qsp 100%

Le mode opératoire est le même que celui de l'exemple 1.

On obtient une composition matifiante qui élimine la brillance de la peau.

### EXEMPLE COMPARATIF

### Crème ne contenant pas de PTFE : émulsion H/E

### Phase huileuse

- Cyclohexadiméthylsiloxane 10%
- Alcool stéarylique 1%
- Mélange de tartrate de dimérystyle/alcool cétéarylique/C₁₂-C₁₅-Pareth-7/PPG-25 laureth-25 commercialisé par la société ENICHEM sous la dénomination COSMACOL PSE® 1,5%

### Phase aqueuse

- Gomme de xanthane 0,2%
- Ammonium polyacryldiméthyltauramide commercialisé par la société HOECHST sous la dénomination HOSTACERIN AMPS® 0,4%
- Hydroxyde de sodium 0,01%
- Glycérine 5%
- Silice
   commercialisée par la société MIYOSHI sous la dénomination SB 150 1,5%
- Aluminium starch octénylsuccinate commercialisé par la société NATIONAL STARCH sous la dénomination DRY-FLO® 3%
- Conservateurs 0,70%
- Eau qsp 100%

Le mode opératoire est le même que celui de l'exemple 1.

On obtient une composition matifiante qui élimine la brillance de la peau.

### Test de matité

On a mesuré la matité obtenue pour les compositions selon l'invention des exemples 1 et 2, comprenant chacune 1,5% de PTFE (en matière active : MA), et pour la comparaison l'exemple comparatif ne comprenant pas de PTFE, mais comprenant 1,5% de silice (MA).

La mesure a été réalisée de la manière suivante: sur un support en caoutchouc, on a étalé la composition à raison de 2 g/cm², on a laissé sécher, puis on a mesuré la réflexion à l'aide d'un gonioréflectomètre, le résultat obtenu étant le rapport R entre la réflexion spéculaire et la réflexion diffuse. La valeur de R est d'autant plus faible que l'effet matifiant est important.

| Composition | Exemple 1 | Exemple 2 | Exemple comparatif |
|---|---|---|---|
| R | 1,97 ± 0,07 | 1,87 ± 0,07 | 2,20 ± 0,08 |

Ces résultats, *in vitro,* montrent qu'avec une concentration en PTFE de 1,5% MA, on obtient un résultat de matité supérieur à celui obtenu avec la formule comparative qui est à 1,5% MA de silice.

### EXEMPLE 3

### Phase huileuse:

- Cyclohexadiméthylsiloxane 10%
- Alcool stéarylique 1%
- Stéarate de glycéryle
   (et) PEG-100 stéarate 2%

### Phase aqueuse:

- Gomme de xanthane 0,2%
- Ammonium polyacryldiméthyltauramide commercialisé par la société HOECHST sous la dénomination HOSTACERIN AMPS® 0,4%
- Hydroxyde de sodium 0,01%
- Glycérine 5%
- Aluminium starch octénylsuccinate commercialisé par la société NATIONAL STARCH sous la dénomination DRY-FLO® 3%
- PTFE en dispersion aqueuse à 40% (taille : 12 µm)
   commercialisé par la société CARROLL SCIENTIFIC sous la dénomination FLUOTRON 200® 3,75%
- Mexoryl SX® 2%
- Nanotitane (oxyde de titane nanométrique) 2%
- triéthanolamine 0,4%
- Conservateurs 0,70%
- Eau qsp 100%

## Revendications

1. Utilisation cosmétique pour le traitement des peaux grasses d'une composition cosmétique matifiante contenant, dans un milieu physiologiquement acceptable, une quantité efficace de polytétrafluoroéthylène (PTFE), le polytétrafluoroéthylène (PTFE) étant sous forme de particules en dispersion dans un milieu aqueux, lesdites particules ayant une dimension moyenne en nombre allant d'environ 0,1 à 30µm, et de préférence de 0,5 à 15µm.

2. Utilisation selon la revendication 1, **caractérisée en ce que** le polytétrafluoroéthylène (PTFE) est présent en une quantité de matière active allant de 0,1 à 30 % en poids, de préférence de 0,5 à 15 % en poids, et mieux de 0,5 à 5 % en poids par rapport au poids total de la composition.

3. Utilisation selon la revendication 1 ou 2, **caractérisée en ce qu'**elle se présente sous forme d'une composition aqueuse.

4. Utilisation selon la revendication 1 ou 2, **caractérisée en ce qu'**elle se présente sous forme d'une émulsion huile-dans-eau (H/E) ou eau-dans-huile (E/H) ou multiple.

5. Utilisation selon la revendication 4, **caractérisée en ce qu'**elle comprend une phase huileuse contenant au moins une huile choisie parmi les huiles d'origine végétale, les huiles minérales, les huiles de synthèse, les huiles de silicone et les huiles fluorées ou fluorosiliconées.

6. Utilisation selon la revendication 5, **caractérisée en ce que** la phase huileuse comprend également des corps gras choisis parmi les acides gras, les alcools gras et les cires.

7. Utilisation selon l'une quelconque des revendications 4 à 6, **caractérisée en ce que** la phase aqueuse représente de 1 à 80% en poids, et de préférence de 30 à 70 % en poids du poids total de la composition, et la phase huileuse représente de 5 à 70% en poids, et de préférence de 10 à 50 % en poids du poids total de la composition.

8. Utilisation selon l'une quelconque des revendications 4 à 7, **caractérisée en ce qu'**elle comprend au moins un émulsionnant choisi parmi les émulsionnants amphotères, anioniques, cationiques ou non ioniques, utilisés seuls ou en mélange.

9. Utilisation selon l'une quelconque des revendications précédentes, **caractérisée en ce qu'**elle comprend un ou plusieurs adjuvants choisis parmi les colorants hydrosolubles ou liposolubles, les pigments, les parfums, les conservateurs, les filtres solaires, les actifs liposolubles ou hydrosolubles, les séquestrants, les hydratants, les ajusteurs de pH (acides ou bases) et les charges.

10. Utilisation selon la revendication 9, **caractérisée en ce que** les filtres solaires sont choisis parmi les filtres UVA, les filtres UVB, les filtres physiques et leurs mélanges.

11. Utilisation selon la revendication 9, **caractérisée en ce que** les actifs sont des actifs utiles pour traiter les peaux grasses, choisis parmi les sels de zinc, et de préférence le gluconate de zinc, les anti-bactériens, le triclosan, le lipacide, l'extrait de clou de girofle, l'octopirox, l'hexamidine, l'acide azélaïque, les actifs anti-acné, et leurs mélanges.

12. Utilisation selon la revendication 9, **caractérisée en ce que** les charges sont choisies parmi la poudre de silice, le talc, les particules de polyamide, les poudres de polyéthylène, les microsphères à base de copolymère acrylique, les poudres expansées, les poudres de matériaux organiques naturels, les microbilles de résine de silicone et leurs mélanges.

13. Utilisation selon l'une quelconque des revendications précédentes, **caractérisée en ce qu'**elle comprend un ou plusieurs gélifiants hydrophiles ou lipophiles choisis parmi les argiles, les gommes polysaccharides et leurs dérivés, les polymères carboxyvinyliques, les polyacrylamides, les polymères d'acide acrylamidométhylpropanesulfonique au moins partiellement réticulés, et leurs mélanges.

14. Procédé de traitement cosmétique des peaux grasses comprenant l'application sur la peau d'une composition cosmétique matifiante contenant, dans un milieu physiologiquement acceptable, une quantité efficace de polytétrafluoroéthylène (PTFE), le polytétrafluoroéthylène (PTFE) étant sous forme de particules en dispersion dans un milieu aqueux, lesdites particules ayant une dimension moyenne en nombre allant d'environ 0,1 à 30µm, et de préférence de 0,5 à 15µm.
